# EUROPÄISCHE PATENTSCHRIFT

(11) **EP 2 482 862 B1**
(45) Veröffentlichungstag und Bekanntmachung des Hinweises auf die Patenterteilung: **15.08.2018**
(21) Anmeldenummer: 10763618.5
(22) Anmeldetag: 30.09.2010
(51) Int. Cl.: A61L 24/00, A61L 24/06, A61L 27/16, A61L 27/54, A61L 27/56

(54) **IMPLANTATMATERIAL SOWIE VERFAHREN ZU DESSEN HERSTELLUNG**
IMPLANT MATERIAL AND A METHOD FOR PRODUCING SAME
MATÉRIAU D'IMPLANT ET PROCÉDÉ DE PRODUCTION

(30) Priorität: 30.09.2009 DE 102009043551
(43) Veröffentlichungstag der Anmeldung: 08.08.2012
(73) Patentinhaber: AAP Biomaterials GmbH, 64807 Dieburg (DE)
(72) Erfinder: BIELENSTEIN, Oliver, 14193 Berlin (DE); DEUSSER, Stefan, 63791 Karlstein (DE); SATTIG, Christoph, 64807 Dieburg (DE); STIRNAL, Volker, 64807 Dieburg (DE)
(74) Vertreter: Herden, Andreas F.
(86) Internationale Anmeldenummer: PCT/EP2010/005952
(87) Internationale Veröffentlichungsnummer: WO 2011/038906

(56) Entgegenhaltungen:
- EP-A1- 1 366 774
- EP-A1- 1 430 913
- RU-C1- 2 306 115

## Beschreibung

Die Erfindung betrifft einen Knochenzement sowie ein Verfahren zu dessen Herstellung.

### Hintergrund der Erfindung

Polymerische Implantatmaterialien sind bekannt. So werden beispielsweise acrylbasierte Knochenzemente verwendet. Insbesondere wird Methylmethacrylat zur Ausbildung eines Polymethylmethacrylat(PMMA)-Knochenzementes verwendet.

Mit derartigen Knochenzementen lassen sich hohe Festigkeiten erreichen, so dass diese auch in der Orthopädie einsetzbar sind. Aber auch in der Vertebroplastie und Kyphoplastie finden Acryl-basierte Knochenzemente Verwendung.

Zumeist werden Thermoplaste verwendet, also Materialien, welche nicht oder nur in sehr geringem Maße quervernetzt sind. Bei in der Medizintechnik bekannten quervernetzenden Zementen gibt es Hinweise auf eine wesentlich geringere Bioverträglichkeit. Derartige Materialien werden daher in der Regel nicht verwendet.

Bekannten Knochenzementen wird zumeist ein Röntgenkontrastmittel zugesetzt. Als Röntgenkontrastmittel sind in der Praxis Zirkonoxid und Bariumoxid verbreitet. Es hat sich herausgestellt, dass diese, in partikulärer Form verwendeten Materialien, häufig zu Einschlüssen von Luft führen. Des Weiteren können die teilweise im submikronbereich vorliegenden Metallpartikel aufgrund ihrer zytotoxischen Wirkung das umliegende Gewebe schädigen. Schließlich können die Metallpartikel an den Kontaktflächen aufgrund ihrer schmirgelnden Wirkung zu Abrasionen und somit zur Bildung von freien Partikeln führen. Diese wirken sich in der Regel sehr negativ auf das umliegende Gewebe aus.

Das Dokument EP 1 366 774 zeigt eine Knochenzementmischung mit einer ein Röntgenkontrastmittel enthaltenden Polymerkomponente. Das Dokument RU 2306115 zeigt ein Implantat für die plastische Chirurgie im Bereich des Auges, welches aus einer Polymerhülle besteht, die mit einem Hydro-Gel befüllt ist, welches u.a. ein Alkalimetallakrylat enthält.

### Aufgabe der Erfindung

Der Erfindung liegt die Aufgabe zugrunde, ein Implantatmaterial mit verbesserten Eigenschaften bereitzustellen.

### Zusammenfassung der Erfindung

Die Aufgabe der Erfindung wird durch ein Verfahren zur Herstellung eines Implantatmaterials sowie durch ein Implantatmaterial nach einem der unabhängigen Ansprüche gelöst.

Bevorzugte Ausführungsformen und Weiterbildungen der Erfindung sind den jeweiligen Unteransprüchen zu entnehmen.

Die Erfindung betrifft ein Verfahren zur Herstellung eines Implantatmaterials, welches als Knochenzement ausgebildet ist. Bei dem Implantatmaterial handelt es sich um ein polymerisches Implantatmaterial, insbesondere um ein Polyacrylat. Gemäß der Erfindung wird mittels eines teilweise polymerisierbaren Metallsalzes ein Polymer, insbesondere ein Polyacrylat, teilweise quervernetzt.

Die Erfinder haben herausgefunden, dass sich über polymerisierbare Metallsalze Quervernetzungen mit verbesserter Biokompatibilität herstellen lassen. Aufgrund der Quervernetzungen kann ein Implantatmaterial mit verbesserten mechanischen Eigenschaften bereitgestellt werden.

Insbesondere konnte durch die Erfindung ein quervernetzter Knochenzement bereit gestellt werden, welcher keine nennenswerten Nebenwirkungen hat. Bekannte Ansätze zur Herstellung quervernetzter Knochenzemente aus Duroplasten oder Elastomeren haben zumeist zu unerwünschten Nebenwirkungen des Implatatmaterials geführt.

Darüber hinaus können über das Metallzentralatom weitere Funktionalitäten, wie beispielsweise die eines Röntgenkontrastmittels, bereitgestellt werden. Vorzugsweise wird zur Quervernetzung ein Metallacrylat verwendet. Es versteht sich, dass zur Herstellung von Quervernetzungen ein Acrylat mit zumindest zwei funktionellen Acrylatgruppen verwendet werden muss. Vorzugsweise wird ein Acrylat mit zumindest drei, besonders bevorzugt zumindest vier Acrylatgruppen verwendet.

Aber auch die Verwendung eines Metalloxalats oder eines Metall(di)maleinats ist denkbar.

Im Sinne der Erfindung wird unter einem Metallsalz auch ein Salz mit einem Metalloxikation, wie beispielsweise ZrO²⁺, angesehen.

Gegenüber der bekannten Verwendung von Zirkonoxid oder Bariumoxid als Zuschlagstoff ist das Metall in dem Polymergerüst eingebunden und liegt somit nicht als partikuläres Oxid vor, welches die genannten nachteiligen Auswirkungen nach sich zieht.

Auch die Verwendung von Metallen für andere Funktionalitäten ist denkbar. So ist beispielsweise denkbar, ein Magnesium- oder Calciumsalz, insbesondere ein Magnesium- oder Calciumacetat als Quervernetzer zu verwenden, um so das Einwachsen zu begünstigen. Auch die Verwendung einer geringen Menge eines Silbersalzes, beispielsweise die Verwendung von Silberacetat, ist denkbar, um so eine bakteriostatische oder bakteriozide Wirkung zu erzielen.

Insbesondere können folgende Metalle Verwendung finden: Ca, Sn, Zr, Fe, Mo, Ag, Mn, Co und/oder Ti.

Das polymerisierbare Metallsalz wird zunächst in einem Monomer, beispielsweise in einem (Meth)acrylat gelöst.

Das Lösen in dem Monomer erfolgt bei einer Ausführungsform der Erfindung derart, dass das Acrylat unmittelbar im Anschluss an dessen Herstellung in einem Monomer gelöst wird. Es hat sich heraus gestellt, dass beispielsweise aus Zirkonacrylat und Methacrylsäure hergestelltes Zirkonacrylatpulver in MMA gut löslich ist. Kommerziell erworbenes Zirkonacrylat löst sich hingegen nicht, möglicherweise da dieses Material mit der Zeit zumindest teilweise polymerisiert, derart, dass eine Löslichkeit im MMA nicht mehr gegeben ist.

Dabei sind Salze von mehrwertigen Metallen, insbesondere von 4-wertigen von Vorteil, da die vier funktionellen Acrylatgruppen das Zentralatom derart abschirmen, dass das Metallacrylat eine so geringe Polarität hat, dass es sich gut in einem Monomer beziehungsweise Prepolymer löst.

Vorzugsweise werden bei der Herstellung 5 bis 90, besonders bevorzugt 10 bis 50 % des polymerisierbaren Metallsalzes verwendet. Diese Angabe bezieht sich auf das Verhältnis des polymerisierbaren Metallsalzes zu den ansonsten verwendeten Monomere, Polymeren oder Prepolymeren. Weitere eventuell verwendete Zuschlagstoffe gehen somit nicht in die Berechnung ein.

Bei einer Weiterbildung der Erfindung werden zunächst teilweise quervernetzte Polymerpartikel hergestellt, welche sodann in einem Monomer teilweise angelöst oder angequollen werden. Anschließend wird aus dem Monomer das Implantatmaterial polymerisiert, wobei die Polymerpartikel mit dem restlichen Polymer vernetzt werden.

Diese Ausführungsform eignet sich insbesondere zur Bereitstellung von Knochenzement. Es wird dabei zunächst, wie vorstehend beschrieben, ein teilweise quervernetztes Polymer, beispielsweise in Form eines Perlpolymerisats hergestellt. Es versteht sich, dass dieses Polymer auch nur teilweise polymerisiert sein kann.

Die Partikel werden in einem zweiten Schritt, welcher beispielsweise in vivo stattfinden kann, mit einem Monomer vernetzt, wobei aufgrund des Anlösens oder Anquellens die Partikel in das sich bildende Polymergerüst eingebunden werden.

Diese Ausführungsform der Erfindung hat zum einen den Vorteil, dass durch die zugesetzten Polymerpartikel eine Paste bereitgestellt werden kann, welche aufgrund ihrer Konsistenz formstabil ist und sich daher gut als Zement verarbeiten lässt. Des Weiteren ist ein hoher Anteil des Implantatmaterials bereits vor dem Einbringen polymerisiert. Die Härtung des Implantatmaterials aufgrund der weiteren Polymerisation durch das Monomer verläuft daher schneller und bei niedrigerer Temperatur.

Bei einer Weiterbildung der Erfindung wird das teilweise quervernetzte Polymer in ein Lösungsmittel gegeben, wobei sich nicht quervernetzte Bestandteile zumindest teilweise herauslösen und so eine poröse Struktur entsteht. Aufgrund der langkettigen Bestandteile, die sich herauslösen, entsteht eine mikroporöse Schwammstruktur mit kanalartigen, zumindest teilweise offenporigen Strukturen.

Der so hergestellte Körper kann eine hohe Elastizität besitzen. Des Weiteren kann die poröse Struktur zur Einbringung einer Wirksubstanz, beispielsweise eines Antibiotikums, verwendet werden.

Herkömmliche wirkstoffhaltige Implantatmaterialien haben oft den Nachteil, dass in der Anfangszeit nach dem Einsetzen der Wirkstoff in hoher Dosis abgegeben wird, sodann aber nur langsam aus dem Implantatmaterial heraus diffundiert. Dieser Nachteil kann durch die zuvor genannte Ausführungsform der Erfindung zumindest verringert werden.

Vorzugsweise werden zwischen 20 und 80 % des Volumens des Implantatmaterials herausgelöst.

Bei einer bevorzugten Ausführungsform der Erfindung wird zur Polymerisation ein Initiator, insbesondere Di-Benzoyl-Peroxid und ein Accelerator, insbesondere Di-Methyl-p-Toluidin, zugesetzt.

Durch Zugabe von Initiator und Accelerator kann, insbesondere zur Verwendung des Implantatmaterials als Knochenzement, bei welcher die Polymerisation zumindest teilweise in vivo stattfindet, die Polymerisation derart beschleunigt werden, dass bereits nach kurzer Zeit eine hinreichende Festigkeit gegeben ist. Des Weiteren kann die Temperatur, ab der eine Polymerisation stattfindet, auf eine nicht gewebeschädigende Temperatur herabgesetzt werden.

Die Erfindung betrifft des Weiteren ein Implantatmaterial, welches mit einem zuvor beschriebenen Verfahren herstellbar ist.

Die Erfindung betrifft einen Knochenzement, welcher ein Polymer umfasst, das über ein zentrales Metallatom quervernetzt ist. Es hat sich herausgestellt, dass über ein zentrales Metallatom bereit gestellte Quervernetzungen nicht zu unerwünschten Nebenwirkungen führen.

Weiter betrifft die Erfindung einen Knochenzement, welcher ein Polyacrylat umfasst, das teilweise über eine Metallacrylatgruppe quervernetzt ist.

Über Metallacrylatgruppen, welche eine teilweise Quervernetzung bewirken, lässt sich ein Implantatmaterial mit verbesserten Eigenschaften bereitstellen. Insbesondere können die mechanischen Eigenschaften verbessert werden.

Bei einer Weiterbildung der Erfindung sind nichtquervernetzte Bestandteile zumindest teilweise herausgelöst. Das Implantat weist eine poröse, insbesondere mikroporöse, Struktur auf.

Bei einer bevorzugten Ausführungsform der Erfindung bildet ein mindestens 4-wertiges Metallatom das Zentralatom der Quervernetzungen.

Das mittlere Molekulargewicht beträgt vorzugsweise zwischen 1x10⁵ und 1,5x10⁶g/mol. Das mittlere Molekulargewicht ist vorzugsweise im Wesentlichen normalverteilt.

Insbesondere ist ein Knochenzement vorgesehen, bei dem Barium, Zirkon oder Titan als Zentralatom der Quervernetzungen eingebunden sind und welches so ein im Polymergerüst molekular eingebundenes Röntgenkontrastmittel aufweist.

Die Erfindung soll im Folgenden anhand von zwei Ausführungsbeispielen näher erläutert werden.

### Beispiel 1

Es wird zunächst ein Zirkonacrylat in (Metyl)methacrylsäure aufgelöst und, beispielsweise unter Temperaturerhöhung, ein teilweise quervernetztes Polymer hergestellt. Das Polymer kann sodann zu einem Perlpolymerisat vermahlen werden oder bereits in einer Suspensionspolymerisation gleich als Perlpolymerisat hergestellt werden. Der Anteil der an Quervernetzungen lässt sich über die Menge an (Methyl)methacrylsäure oder Methyl(acrylatsäure) steuern. So führt ein Überschuss an zum Beispiel Methacrylsäure zu einem geringeren Anteil an Quervernetzungen.

Es versteht sich, dass bei der Polymerisation noch weitere Stoffe, wie beispielsweise Initiatoren oder Acceleratoren verwendet werden können.

Zum Anmischen eines Knochenzements wird das Perlpolymerisat mit Metylmethacrylat unter Zugabe von Dimethyl-p-Toluidin als Accelerator und Di-Benzoyl-Peroxid als Initiator zu einer Paste vermischt.

Die Paste wird sodann als Knochenzement verarbeitet und beispielsweise mittels einer Spritze oder von Hand in Defektstellen eingebracht.

Durch das Metylmethacrylat werden nicht quervernetzte Bestandteile des Perlpolymerisats angelöst und das Perlpolymerisat in das Polymergerüst eingebunden. Aufgrund des in dem Polymergerüst eingebundenen Zirkons umfasst der Knochenzement ein Röntgenkontrastmittel, so dass auf partikuläre Metalloxidzusätze verzichtet werden kann.

### Beispiel 2

Zunächst wird, ähnlich wie vorstehend beschrieben, ein Metallacrylat in Metylmethacrylat gelöst und sodann ein teilweise quervernetztes Polymer hergestellt. Beispielsweise mittels Methacrylsäure als Lösungsmittel werden sodann nicht quervernetzte Bestandteile zumindest teilweise herausgelöst.

Es entsteht ein poröser Formkörper.

## Patentansprüche

1. Verfahren zur Herstellung eines Implantatmaterials, welches als Knochenzement ausgebildet ist, wobei ein polymerisierbares Metallacrylat in einem Monomer gelöst wird und sodann ein quervernetztes Polymer hergestellt wird.

2. Verfahren zur Herstellung eines Implantatmaterials nach dem vorstehenden Anspruch, **dadurch gekennzeichnet, dass** ein Ba, Sr, Mg, Zn, Sn, Cu, Zr, Fe, Cr, Mo, Mn, Co und/oder Ti-acrylat verwendet wird.

3. Verfahren zur Herstellung eines Implantatmaterials nach einem der vorstehenden Ansprüche, **dadurch gekennzeichnet, dass** ein Metallacrylat mit zumindest einem vierwertigen Zentralatom verwendet wird.

4. Verfahren zur Herstellung eines Implantatmaterials nach einem der vorstehenden Ansprüche, **dadurch gekennzeichnet, dass** bei der Herstellung 10 bis 50 % des polymerisierbaren Metallacrylats verwendet werden.

5. Verfahren zur Herstellung eines Implantatmaterials nach einem der vorstehenden Ansprüche, **dadurch gekennzeichnet, dass** zunächst teilweise quervernetzte Polymerpartikel hergestellt werden, welche sodann in einem Monomer angelöst oder angequollen werden und aus dem Monomer das Implantatmaterial polymerisiert wird, wobei die Polymerpartikel mit dem restlichen Polymer vernetzt werden.

6. Verfahren zur Herstellung eines Implantatmaterials nach einem der vorstehenden Ansprüche, **dadurch gekennzeichnet, dass** das teilweise quervernetzte Polymer in ein Lösungsmittel gegeben wird, wobei sich nicht quervernetzte Bestandteile zumindest teilweise herauslösen und eine poröse Struktur entsteht.

7. Verfahren zur Herstellung eines Implantats nach dem vorstehenden Anspruch, **dadurch gekennzeichnet, dass** zwischen 20 und 80 % des Volumens des Implantats herausgelöst wird.

8. Verfahren zur Herstellung eines Implantatmaterials nach einem der vorstehenden beiden Ansprüche, **dadurch gekennzeichnet, dass** die poröse Struktur als Wirkstoffträger, insbesondere für ein Bakteriozid, verwendet wird.

9. Verfahren zur Herstellung eines Implantatmaterials nach einem der vorstehenden Ansprüche, **dadurch gekennzeichnet, dass** zur Polymerisation ein Initiator, insbesondere Di-Benzoyl-Peroxid und ein Accelerator, insbesondere Dimetyl-p-Toluidin, zugegeben wird.

10. Verfahren zur Herstellung eines Implantatmaterials nach einem der vorstehenden Ansprüche, **dadurch gekennzeichnet, dass** das Lösen in dem Momomer derart erfolgt, dass das Acrylat unmittelbar im Anschluss an dessen Herstellung in dem Momomer gelöst wird.

11. Implantatmaterial, herstellbar nach einem der vorstehenden Ansprüche, umfassend ein Polyacrylat, welches teilweise über eine Metallacrylatgruppe quervernetzt ist.

12. Implantatmaterial gemäß Anspruch 11, **dadurch gekennzeichnet, dass** nicht quervernetzte Bestandteile zumindest teilweise herausgelöst sind und/oder dass das Implantat eine poröse, insbesondere mikroporöse, Struktur aufweist und/oder dass Ba, Sr, Mg, Zn, Sn, Zr, Fe, Mo, Mn, Co oder Ti oder ein Oxid davon das Zentralatom der Quervernetzungen bildet.

13. Implantatmaterial gemäß Ansprüchen 11-12, **dadurch gekennzeichnet, dass** ein mindestens 4-wertiges Metallatom das Zentralatom der Quervernetzungen bildet.

## Claims

1. A method for producing an implant material in the form of a bone cement, wherein a polymerizable metal acrylate is dissolved in a monomer, and then a crosslinked polymer is produced.

2. The method for producing an implant material according to the preceding claim, **characterised in that** one of Ba-, Sr-, Mg-, Zn-, Sn-, Cu-, Zr-, Fe-, Cr-, Mo-, Mn-, Co- and/or Ti-acrylate is used.

3. The method for producing an implant material according to any one of the preceding claims, **characterised in that** a metal acrylate with at least one tetravalent central atom is used.

4. The method for producing an implant material according to any one of the preceding claims, **characterised in that** 10 % to 50 % of the polymerizable metal acrylate is used for the producing.

5. The method for producing an implant material according to any one of the preceding claims, **characterised in that** initially partially crosslinked polymer particles are prepared, which are then caused to dissolve or swell to a certain extent in a monomer, and the implant material is polymerized from the monomer, the polymer particles being crosslinked with the remaining polymer.

6. The method for producing an implant material according to any one of the preceding claims, **characterised in that** the partially crosslinked polymer is added to a solvent, whereby non-crosslinked components are dissolved out at least partially and a porous structure is formed.

7. The method for producing an implant according to the preceding claim, **characterised in that** between 20 % and 80 % of the volume of the implant is dissolved out.

8. The method for producing an implant material according to any one of the preceding two claims, **characterised in that** the porous structure is used as a pharmaceutical drug carrier, in particular for a bactericide.

9. The method for producing an implant material according to any one of the preceding claims, **characterised in that** an initiator, in particular di-benzoyl peroxide, and an accelerator, in particular dimethyl-p-toluidine, are added for the polymerization.

10. The method for producing an implant material according to any one of the preceding claims, **characterised in that** the dissolving in the monomer takes place in a manner so that the acrylate is dissolved in the monomer immediately after having been produced.

11. An implant material, producible according to any one of the preceding claims, comprising a polyacrylate which is partially crosslinked via a metal acrylate group.

12. The implant material according to claim 11, **characterised in that** non-crosslinked components are at least partially dissolved out, and/or that the implant has a porous, in particular microporous structure, and/or that Ba, Sr, Mg, Zn, Sn, Zr, Fe, Mo, Mn, Co, or Ti or an oxide thereof forms the central atom of the crosslinks.

13. The implant material according to any one of claims 11 to 12, **characterised in that** an at least tetravalent metal atom forms the central atom of the crosslinks.

## Revendications

1. Procédé de fabrication d'un matériau d'implant, qui est sous forme d'un ciment osseux, dans lequel un acrylate métallique polymérisable est dissous dans un monomère et un polymère réticulé est ainsi produit.

2. Procédé de fabrication d'un matériau d'implant selon la revendication précédente, **caractérisé en ce qu'**un acrylate de Ba, Sr, Mg, Zn, Sn, Cu, Zr, Fe, Cr, Mo, Mn, Co et/ou Ti est utilisé.

3. Procédé de fabrication d'un matériau d'implant selon l'une des revendications précédentes, **caractérisé en ce qu'**un acrylate métallique comportant au moins un atome central quadrivalent est utilisé.

4. Procédé de fabrication d'un matériau d'implant selon l'une des revendications précédentes, **caractérisé en ce que** 10 à 50 % de l'acrylate métallique polymérisable sont utilisés lors de la fabrication.

5. Procédé de fabrication d'un matériau d'implant selon l'une des revendications précédentes, **caractérisé en ce que** des particules polymères partiellement réticulées sont d'abord fabriquées qui sont ensuite dissoutes ou gonflées dans un monomère, et le matériau d'implant est polymérisé à partir du monomère, les particules polymères étant réticulées avec le polymère restant.

6. Procédé de fabrication d'un matériau d'implant selon l'une des revendications précédentes, **caractérisé en ce que** le polymère partiellement réticulé est versé dans un solvant, les composants non réticulés se séparant au moins partiellement et une structure poreuse en résultant.

7. Procédé de fabrication d'un implant selon la revendication précédente, **caractérisé en ce qu'**entre 20 et 80 % du volume de l'implant se séparent.

8. Procédé de fabrication d'un matériau d'implant selon l'une des deux revendications précédentes, **caractérisé en ce que** la structure poreuse est utilisée comme support de substances actives, notamment pour un bactériocide.

9. Procédé de fabrication d'un matériau d'implant selon l'une des revendications précédentes, **caractérisé en ce qu'**un initiateur, notamment du di-peroxyde de benzoyle et un accélérateur, notamment de la diméthyl-p-toluidine, sont ajoutés pour la polymérisation.

10. Procédé de fabrication d'un matériau d'implant selon l'une des revendications précédentes, **caractérisé en ce que** la dissolution dans le monomère s'effectue de sorte que l'acrylate soit dissous dans le monomère directement à la suite de sa fabrication.

11. Matériau d'implant, pouvant être fabriqué d'après l'une des revendications précédentes, comprenant un polyacrylate, qui est partiellement réticulé par le biais d'un groupe acrylate métallique.

12. Matériau d'implant selon la revendication 11, **caractérisé en ce que** les composants non réticulés se séparent au moins partiellement et/ou **en ce que** l'implant présente une structure poreuse, notamment microporeuse, et/ou **en ce que** Ba, Sr, Mg, Zn, Sn, Zr, Fe, Mo, Mn, Co ou Ti ou un oxyde de ceux-ci forme l'atome central des réticulations.

13. Matériau d'implant selon les revendications 11 et 12, **caractérisé en ce qu'**un atome métallique au moins quadrivalent forme l'atome central des réticulations.
